# EUROPÄISCHE PATENTANMELDUNG

(11) **EP 3 564 680 A1**
(43) Veröffentlichungstag der Anmeldung: **06.11.2019**
(21) Anmeldenummer: 19175095.9
(22) Anmeldetag: 08.01.2014
(51) Int. Cl.: G01N 35/00, G01N 35/02, G01N 35/10

(54) **PROBENVERARBEITUNGSSYSTEM MIT DOSIERVORRICHTUNG UND THERMOCYCLER**

(30) Priorität: 09.01.2013 DE 102013200193
(62) Teilanmeldung aus: 14150467.0
(71) Anmelder: Hamilton Bonaduz AG, 7402 Bonaduz (CH)
(72) Erfinder: Gajewski, Martin, 42799 Leichlingen (DE); Arangio, Mario, 7000 Chur (CH)
(74) Vertreter: Müller Hoffmann & Partner

(57) **Zusammenfassung**

Die Erfindung betrifft Probenverarbeitungssystem und Verfahren zum Verarbeiten von chemischen oder biologischen Proben, insbesondere Körperflüssigkeiten wie Blut, Speichel, Sekrete und dergleichen oder Gewebeproben, insbesondere zur Verarbeitung von Proben für eine DNA-Analyse mittels PCR, umfassend eine Dosiervorrichtung mit einer Aufnahmeplatte (12), wobei die Aufnahmeplatte (12) in einer durch eine erste und eine zweite Richtung (X, Y) definierten Ebene im Wesentlichen horizontal angeordnet ist;
mit wenigstens einem Arbeitsarm (14, 16, 18), der relativ zur Aufnahmeplatte (12), vorzugsweise entlang einem ersten Rand (13) der Aufnahmeplatte (12), in der ersten Richtung (X) beweglich ist, und wobei sich der Arbeitsarm (14, 16, 18) in der zweiten Richtung (Y) über die Aufnahmeplatte (12) erstreckt, wobei am Arbeitsarm (14, 16, 18) wenigstens eine in der zweiten Richtung (Y) bewegliche Pipettiervorrichtung (30) angebracht ist, wobei die Pipettiervorrichtung (30) wenigstens einen in einer zur ersten und zweiten Richtung (X, Y) orthogonalen dritten Richtung (Z) beweglichen Pipettierkanal (32) mit einer daran angebrachten oder anbringbaren Pipettierspitze (33) umfasst und wobei die Pipettiervorrichtung (30) dazu eingerichtet ist, Flüssigkeit in bzw. aus einem Probenbehälter zu dispensieren bzw. zu aspirieren; und wenigstens einen Thermocycler (50, 52), der im Bereich der Aufnahmeplatte (12) vorgesehen ist. Erfindungsgemäß wird vorgeschlagen, dass der Thermocycler (50, 52) derart an der Aufnahmeplatte (12) angeordnet ist, dass die Pipettiervorrichtung (30) oberhalb von Probenbehältern, die sich im Thermocycler (50, 52) befinden, in eine einen Pipettiervorgang ermöglichende Stellung beweglich ist. Das Öffnen/Schließen eines Verschlusselements (54) des Thermocyclers erfolgt in Abhängigkeit von Schritten eines PCR-Prozesses und das Pipettieren von Komponenten in Probenbehälter im Thermocycler erfolgt in Abhängigkeit der Stellung des Verschlusselements (54).

## Beschreibung

Die vorliegende Erfindung betrifft ein Probenverarbeitungssystem zum Verarbeiten von chemischen oder biologischen Proben, insbesondere Körperflüssigkeiten wie Blut, Speichel, Sekrete und dergleichen oder Gewebeproben, insbesondere zur Verarbeitung von Proben für eine DNA-Analyse mittels PCR, sowie ein Verfahren zum Steuern eines solchen Probenverarbeitungssystems.

Das Probenverarbeitungssystem umfasst
eine Dosiervorrichtung
- mit einer Aufnahmeplatte, wobei die Aufnahmeplatte in einer durch eine erste und eine zweite Richtung (X, Y) definierten Ebene im Wesentlichen horizontal angeordnet ist; und
- mit wenigstens einem Arbeitsarm, der relativ zur Aufnahmeplatte (14), vorzugsweise entlang einem ersten Rand der Aufnahmeplatte, in der ersten Richtung (X) beweglich ist, und wobei sich der Arbeitsarm in der zweiten Richtung (Y) über die Aufnahmeplatte erstreckt, wobei am Arbeitsarm wenigstens eine in der zweiten Richtung (Y) bewegliche Pipettiervorrichtung angebracht ist, wobei die Pipettiervorrichtung wenigstens einen in einer zur ersten und zweiten Richtung (X, Y) orthogonalen dritten Richtung (Z) beweglichen Pipettierkanal mit einer daran angebrachten oder anbringbaren Pipettierspitze umfasst und wobei die Pipettiervorrichtung dazu eingerichtet ist, Flüssigkeit in bzw. aus einem Probenbehälter zu dispensieren bzw. zu aspirieren;
und wenigstens einen Thermocycler, der im Bereich der Aufnahmeplatte vorgesehen ist.

Ein derartiges Probenverarbeitungssystem ist beispielsweise aus der WO 99/260070 A2 bekannt.

Ferner ist ein weiteres System unter der Bezeichnung "epMotion 5075 MC" der Firma Eppendorf AG bekannt (siehe http://www.eppendorf.com/int/index.php?pb=25118564b687acb3&page=3&action=epmoti on&contentid=3&page=8).

Bei beiden bekannten Probenverarbeitungssystemen ist im Bereich der Aufnahmeplatte, auf welcher Probenbehälterträger zum Pipettieren, ein Abfallbehälter für gebrauchte Pipettierspitzen, eine Misch-/Rüttelvorrichtung und dergleichen angeordnet sein können, zusätzlich ein Thermocycler angeordnet. Dieser Thermocycler kann allerdings nur mit bereits befüllten Probenbehältern auf ihren Probenbehälterträgern (PCR-Platten oder dergleichen) automatisiert bestückt werden mittels eines Greifers. In beiden bekannten Probenverarbeitungssystemen wird Wert darauf gelegt, dass der Thermocycler und die Pipettiereinrichtung durch räumlich getrennte Arbeitsstationen ausgebildet sind. Normalerweise werden die Probenbehälter bzw. Probenbehälterträger für die Durchführung von PCR-Prozessschritten bereits abgedichtet in den Thermocycler gestellt und anschließend wird der Thermocycler mittels eines Deckels verschlossen. Das Zuführen oder Entnehmen von Probenflüssigkeit ist während der Prozessierschritte im Thermocycler nicht möglich.

Aufgabe der Erfindung ist es, ein bekanntes Probenverarbeitungssystem derart weiterzuentwickeln, dass eine flexiblere Handhabung während des Prozessierens von Proben ermöglicht wird.

Erfindungsgemäß wird hierzu vorgeschlagen, dass der Thermocycler derart an der Aufnahmeplatte angeordnet ist, dass die Pipettiervorrichtung oberhalb von Probenbehältern, die sich im Thermocycler befinden, in eine einen Pipettiervorgang ermöglichende Stellung beweglich ist.

Hierdurch wird es in vorteilhafter Weise möglich, während und/oder zwischen Prozessierschritten, die im Thermocycler durchgeführt werden müssen, Pipettiervorgänge durchzuführen, ohne dass hierzu die Probenbehälter (auch Wells genannt) bzw. der zugehörige Probenbehälterträger (beispielsweise eine sogenannte PCR-Platte mit darin angeordneten Wells) aus dem Thermocycler entnommen und an eine Pipettierposition der Aufnahmeplatte transportiert werden muss. Es ist somit sehr einfach möglich, zu einer im Thermocycler erwärmten Probenflüssigkeit (auch als PCR-Reaktionsgemisch bekannt) eine weitere Reaktionskomponente beizugeben, ohne, dass die Proben abkühlen und nach einem außerhalb des Thermocyclers stattfindenden Pipettierschritt erneut auf die gewünschte Temperatur erwärmt werden müssen. Es ergibt sich insgesamt eine vereinfachte Handhabung, insbesondere im Rahmen von PCR-Prozessen, weil alle für PCR-Prozesse erforderlichen Zyklen im Thermocycler abgearbeitet werden können und für bestimmte Prozessschritte erforderliche Komponenten durch die Pipettiervorrichtung den Proben zugegeben werden können, ohne die Probenbehälter bzw. deren Probebehälterträger aus dem Thermocycler entnehmen zu müssen. Dies führt zu einer verbesserten Automatisierung der Prozessierung und damit verbunden einer Zeitersparnis, insbesondere aufgrund des Wegfalls von Transportschritten zwischen dem Thermocycler und einer Pipettierposition auf der Aufnahmeplatte und aufgrund des Wegfalls von längeren Temperierungsschritten, wenn ein Probenbehälterträger (PCR-Platte) aus dem Thermocycler entnommen und später wieder eingesetzt werden muss.

Selbstverständlich kann die Pipettiervorrichtung während des Abarbeitens von Zyklen durch den Thermocycler zeitlich parallel andere Pipettiervorgänge außerhalb des Thermocyclers ausführen, wie etwa Vorbereiten einer nächsten Charge von Probenbehältern für einen nächsten Prozess im Thermocycler.

Es wird weiter vorgeschlagen, dass das Probenverarbeitungssystem ferner eine Steuereinrichtung umfasst, die dazu eingerichtet ist, die Funktionen der Dosiervorrichtung, wie etwa Bewegung in den drei Richtungen oder/und Aspirieren bzw. Dispensieren von Flüssigkeit mittels der Pipettiervorrichtung, und die Funktionen des Thermocyclers , wie etwa Durchführung von Zyklen im Rahmen eines PCR-Prozesses, anzusteuern bzw. zu regeln. Eine derartige Steuereinrichtung kann durch einen Computer mit entsprechenden Steuerprogrammen realisiert sein. Denkbar ist auch, dass die Steuereinrichtung mehrere miteinander vernetzte Computer umfasst, beispielsweise einen Prozessor für die Pipettiereinrichtung und einen Prozessor für den Thermocycler.

Gemäß einer Ausführungsvariante kann der Thermocycler auf der Aufnahmeplatte angeordnet sein, wobei ein höchster Punkt des Thermocyclers unterhalb eines maximal möglichen Abstands zwischen einem unteren Ende der wenigstens einen Pipettierspitze und der Aufnahmeplatte liegt, wobei der Abstand durch eine Bewegung der Pipettiervorrichtung in der dritten Richtung (Z) ermöglicht wird.

Alternativ dazu wird vorgeschlagen, dass der Thermocycler in einem in der Aufnahmeplatte ausgeführten Ausschnitt eingelassen ist, derart, dass eine Unterseite des Thermocyclers unterhalb der Aufnahmeplatte liegt, wobei ein höchster Punkt des Thermocyclers unterhalb eines maximal möglichen Abstands zwischen einem unteren Ende der wenigstens einen Pipettierspitze und der Aufnahmeplatte liegt, wobei der Abstand durch eine Bewegung der Pipettiervorrichtung in der dritten Richtung (Z) ermöglicht wird.

Für beide Ausführungsformen ist es bevorzugt, dass der maximale Abstand zwischen dem unteren Ende der wenigstens einen Pipettierspitze und der Aufnahmeplatte etwa 100mm bis 180mm, vorzugsweise etwa 125mm bis 150mm beträgt.

Der maximale Abstand zwischen der Aufnahmeplatte und dem unteren Ende der wenigstens einen Pipettierspitze kann auch als maximale Überfahrhöhe bezeichnet werden. Ist ein im Bereich der Aufnahmeplatte angeordneter Gegenstand höher als dieser maximale Abstand, stellt er ein Hindernis für die Pipettiervorrichtung dar, die dann in diesen Bereich nicht oder nur dann bewegt werden kann, wenn keine Pipettierspitze am Pipettierkanal angebracht ist. Derartige Gegenstände können zwar auf der Aufnahmeplatte angeordnet werden, müssen dann aber in der Bewegungssteuerung der Pipettiervorrichtung so berücksichtigt werden, dass Kollisionen vermieden werden.

Bisher bekannte Thermocycler weisen in der Regel eine maximale Bauhöhe auf, die größer ist als der erwähnte maximale Abstand zwischen der Pipettierspitze und der Aufnahmeplatte. Ein bestehender Thermocycler kann daher bevorzugt gemäß der Ausführungsform mit einem Ausschnitt in der Aufnahmeplatte verwendet werden, in den der bekannte Thermocycler eingelassen wird.

Bevorzugt wird ein auf die Pipettiervorrichtung und den erwähnten maximalen Abstand zwischen der Pipettierspitze und der Aufnahmeplatte angepasster Thermocycler eingesetzt, der ein die Dosiervorrichtung ergänzendes Modul darstellt und, wie andere Komponenten der Dosiervorrichtung, wie etwa Abfallbehälter, Mischvorrichtung und dergleichen, an einer gewünschten Position auf der Aufnahmeplatte angeordnet werden kann.

Am Thermocycler ist vorzugsweise ein deckelartiges Verschlusselement zum Abdecken von im Thermocycler befindlichen Probenbehältern vorgesehen, wobei das Verschlusselement im Wesentlichen parallel zur Aufnahmeplatte relativ zum Thermocycler beweglich ist zwischen einer das Innere des Thermocyclers freigebenden Öffnungsstellung und einer das Innere des Thermocyclers abdeckenden Schließstellung. Ein derartiges Verschlusselement ragt in seiner Öffnungsstellung nicht nach oben hin weg in den Bereich, in dem sich die Pipettiervorrichtung und die daran angebrachten Pipettierspitzen bewegen. Der freie Zugang der Pipettiervorrichtung zu Probenbehältern im Thermocycler wird somit unterstützt.

Es wird ferner vorgeschlagen, dass die Steuereinrichtung derart eingerichtet ist, dass das Verschlusselement des Thermocyclers in Abhängigkeit der Relativstellung der Pipettiervorrichtung zum Thermocycler in seine Öffnungs- oder Schließstellung bewegt wird, vorzugsweise dass das Verschlusselement dann in seine Öffnungsstellung bewegt wird, wenn mittels der Pipettiervorrichtung ein Pipettiervorgang an Probenbehältern im Thermocycler durchgeführt wird und dass das Verschlusselement in seine Schließstellung bewegt wird, nachdem der Pipettiervorgang an Probenbehältern im Thermocycler beendet ist.

Eine derartige Ansteuerung ermöglicht das Öffnen des Thermocyclers erst kurz vor dem eigentlichen Pipettiervorgang, so dass insbesondere kühlende Temperatureinflüsse von der Umgebung verringert werden können. Es ist auch denkbar, dass nach einem Pipettierschritt, beispielsweise dem gleichzeitigen Dispensieren einer Komponente in mehrere Probenbehälter, das Verschlusselement kurzzeitig wieder in seine Schließstellung gebracht wird bis mittels der Pipettiervorrichtung die gebrauchten Pipettierspitzen im Abfallbehälter entsorgt, neue (ungebrauchte) Pipettierspitzen geholt und die zuzugebende Komponente aus einem Vorratsbehälter aspiriert worden ist. Sobald die Pipettiervorrichtung mit ungebrauchten und mit der Komponente gefüllten Pipettierspitzen auf dem Weg zum Thermocycler ist, kann das Verschlusselement wieder in seine Öffnungsstellung bewegt werden, so dass in eine nächste Reihe von mehreren Probenbehältern die Komponente dispensiert werden kann. Es ist natürlich auch denkbar, dass das Verschlusselement jeweils nur soweit geöffnet wird, dass diejenigen Probenbehälter zugänglich sind, in die eine Komponente dispensiert oder aus denen Probenflüssigkeit aspiriert werden soll. Auch eine derartige teilweise Freigabe der Probenbehälter kann als Öffnungsstellung verstanden werden. Dies kann beispielsweise durch ein zweiteiliges Verschlusselement erreicht werden, wobei zwischen den beiden beweglichen Verschlusselementteilen bei Bedarf eine Lücke belassen wird und in der Lücke mehrere Probenbehälter frei zugänglich sind.

Das Probenverarbeitungssystem umfasst vorzugsweise eine relativ zur Aufnahmeplatte in der ersten, zweiten und dritten Richtung bewegliche Greifeinrichtung, die von der Steuereinrichtung angesteuert wird und dazu eingerichtet ist, Probenbehälterträger zu ergreifen und an gewünschte Positionen auf der Aufnahmeplatte zu transportieren.

Hierzu wird weiterbildend vorgeschlagen, dass die Greifeinrichtung dazu eingerichtet ist, Probenbehälter im bzw. aus dem Thermocycler abzusetzen bzw. zu entnehmen.

Dabei wird auch daran gedacht, dass die Greifeinrichtung an dem Arbeitsarm angeordnet ist oder an einem weiteren relativ zur Aufnahmeplatte beweglichen Arbeitsarm vorgesehen ist.

Es wird darauf hingewiesen, dass am Probenverarbeitungssystem selbstverständlich entsprechende Antriebskomponenten inklusive zugehörige Getriebe, wie etwa Elektromotoren, hydraulische oder pneumatische Antriebe und der gleichen vorgesehen sind, um die gewünschten Bewegungen der Dosiervorrichtung, der Greifeinrichtung, des Verschlusselements des Thermocyclers und dergleichen zu ermöglichen. Diese Antriebskomponenten werden bevorzugt von der Steuereinrichtung angesteuert.

Das der Erfindung zugrunde liegende Problem wird ferner gelöst durch ein Verfahren zum Steuern eines oben vorgestellten Probenverarbeitungssystems, wobei das Verfahren folgende Schritte umfasst:
- Öffnen des Verschlusselements eines ersten Thermocyclers;
- Absetzen eines Probenbehälter enthaltenden Probenbehälterträgers im ersten Thermocycler mittels der Greifeinrichtung, wobei die Probenbehälter mit Probenflüssigkeit befüllt oder unbefüllt sind;
- Gegebenenfalls Befüllen der Probenbehälter mit zu analysierender Probenflüssigkeit mittels der Pipettiervorrichtung;
- vorzugsweise Befüllen der Probenbehälter mit weiteren für die Analyse erforderlichen Komponenten, wie etwa Primer, Polymerase, Wasser und dergleichen mittels der Pipettiervorrichtung;
- Durchführen eines PCR-Prozesses, wobei in Abhängigkeit der durchzuführenden Schritte des PCR-Prozesses das Verschlusselement des ersten Thermocyclers geöffnet oder geschlossen wird und wobei in Abhängigkeit der Stellung des Verschlusselements wenigstens eine weitere Komponente in die im ersten Thermocycler befindlichen Probenbehälter dispensiert wird mittels der Pipettiervorrichtung.

Das vorgeschlagene Verfahren ermöglicht das Pipettieren von Flüssigkeiten bzw. (Reaktions-)Komponenten während sich die Probenbehälter in einem Thermocycler befinden, der automatisiert mit dem Verschlusselement geöffnet oder geschlossen werden kann. Dabei erfolgen Pipettiervorgänge bei geöffnetem Verschlusselement. Es ist selbstverständlich, dass das Verschlusselement auch zur Entnahme eines Probenträgerbehälters nach Abschluss eines PCR-Prozesses geöffnet wird, ohne dass dann ein Pipettiervorgang stattfindet. Das heisst, die Abhängigkeit zwischen der Stellung des Verschlusselements und einem Pipettiervorgang ist nicht so zu verstehen, dass bei jedem Öffnen des Verschlusselements auch ein Pipettiervorgang stattfinden muss. Vielmehr kann bei geöffnetem Verschlusselement ein Pipettiervorgang stattfinden, wenn dies für den PCR-Prozess erforderlich ist.

Weiter kann das Verfahren das vorzugsweise automatische Anordnen oder Entfernen eines die Probenbehälter abdichtenden Dichtungselements umfassen, wie etwa Dichtungsmatte oder dergleichen auf dem Probenbehälterträger in Abhängigkeit des PCR-Prozesses und der Stellung des Verschlusselements.

Ein derartiges Dichtungselement verhindert das Verdampfen von Probenflüssigkeit bzw. leicht flüchtigen Anteilen einer zu analysierenden Probe, wenn diese während des PCR-Prozesses auf Temperaturen bis zu etwa 95° C. erwärmt wird. Ein derartiges Dichtungselement ist vor einem Pipettiervorgang zu entfernen, was bevorzugt automatisiert erfolgt, beispielsweise mittels der Greifeinrichtung oder einer dafür speziell ausgebildeten Vorrichtung, die im Probenverarbeitungssystem vorgesehen ist.

Nach Abschluss eines bestimmten Schritts des PCR-Prozesses kann das Verschlusselement des ersten Thermocyclers geöffnet werden, das vorzugsweise auf den Probenbehältern angeordnete Dichtungselement kann entfernt werden und aus den Probenbehältern prozessierte Probenflüssigkeit kann mittels der Pipettiervorrichtung aspiriert werden und in weitere, insbesondere leere Probenbehälter außerhalb des ersten Thermocyclers dispensiert werden, wobei die anderen Probenbehälter bevorzugt in einem Probenbehälterträger angeordnet sind, der vorzugsweise in einem zweiten insbesondere benachbarten Thermocycler oder auf der Aufnahmeplatte angeordnet ist.

Das Verfahren kann ferner umfassen: das Dispensieren einer weiteren Komponente in die im ersten oder/und zweiten Thermocycler enthaltenen Probenbehälter mittels der Pipettiervorrichtung, nach dem Pipettiervorgang das Anordnen des Dichtungselements auf den Probenbehältern, das Schließen des Verschlusselements des ersten oder/und zweiten Thermocyclers und das Durchführen von weiteren Schritten des PCR-Prozesses.

Nachfolgend wird die Erfindung anhand einer Ausführungsform beispielhaft und nicht einschränkend beschrieben.
- Fig. 1: zeigt in vereinfachter schematischer Perspektivdarstellung eine Ausführungsform eines Probenverarbeitungssystems.
- Fig. 2: zeigt das Probenverarbeitungssystem in einer Aufrissdarstellung von vorne in der zweiten (Y-) Richtung.
- Fig. 3: zeigt eine vergrößerte Teildarstellung der Fig. 2 entsprechend dem dortigen stricht-punktierten Bereich III.

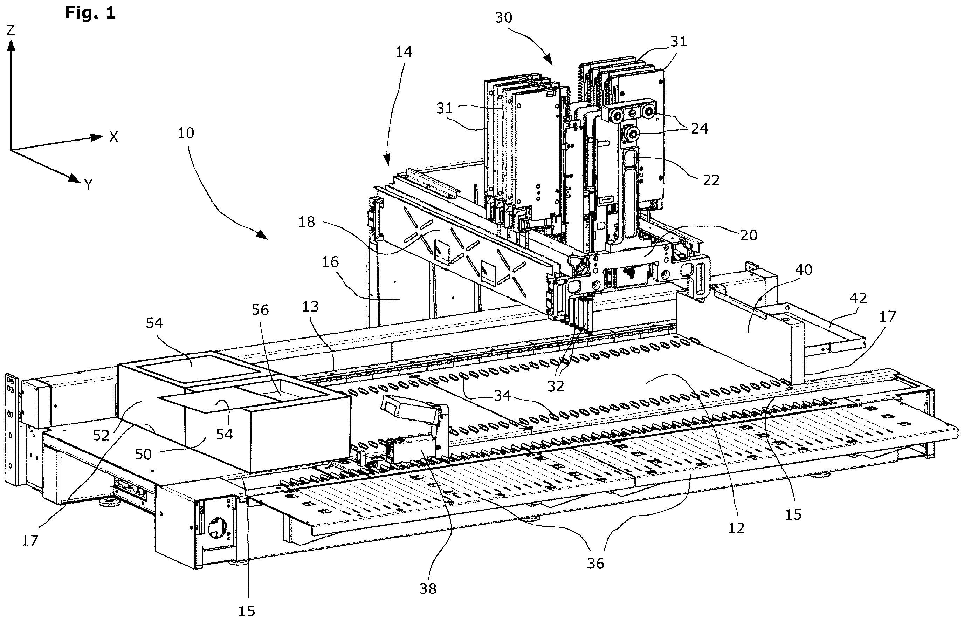

In Fig. 1 ist eine Ausführungsform des Probenverarbeitungssystems 10 dargestellt. Es umfasst eine Aufnahmeplatte 12 und einen entlang eines hinteren Rands 13 der Aufnahmeplatte in einer ersten Richtung X beweglichen Arbeitsarm 14. Der Arbeitsarm 14 umfasst einen sich in einer dritten Richtung Z erstreckenden Träger 16 und einen am Träger 16 befestigten Ausleger 18. Der Ausleger 18 erstreckt sich in einer zweiten Richtung Y über die Aufnahmeplatte 12. An einem vorderen Ende 20 des Auslegers 18 ist eine Aufhängung 22 mit Rollen 24 ersichtlich, die in entsprechenden hier nicht dargestellten Führungen aufgenommen sind.

Am Arbeitsarm 12, insbesondere an seinem Ausleger 18 sind mehrere zur gesamten Pipettiervorrichtung 30 zugehörige Pipettiereinheiten 31 vorgesehen mit einem jeweiligen Pipettierkanal 32. In der Fig. 1 sind in X-Richtung auf beiden Seiten des Auslegers 18 aus Gründen der Übersichtlichkeit jeweils nur vier Pipettiereinheiten 31 dargestellt. In der Regel können auf einer Seite des Auslegers 18 bis zu acht Pipettiereinheiten 31 vorgesehen sein. Die dargestellten Pipettierkanäle 32, zumindest die in Y-Richtung vorderen vier sind ohne zugehörige Pipettiereinheit 31 dargestellt. Die Anzahl Pipettiereinheiten 31 richtet sich nach einer gewünschten Konfiguration der Pipettiervorrichtung 30 bzw. des gesamten Probenverarbeitungssystems 10; es ist auch denkbar, dass nur auf einer Seite des Auslegers 18 eine Pipettiervorrichtung 30 vorgesehen ist. Die Pipettiereinheiten 31 sind entlang des Auslegers 18 in Y-Richtung beweglich und die Pipettierkanäle 32 sind zusätzlich in Z-Richtung beweglich.

Auf der Aufnahmeplatte 12 sind in regelmäßigen Abständen Führungselemente 34 angebracht, an denen Probenbehälterträger (z.B. Träger für Reagenzröhrchen, PCR-Platten etc.), Träger für ungebrauchte Einwegpipettierspitzen, Vorratsbehälter für zu pipettierende Reaktionskomponenten und dergleichen spalten-/zeilenartig angeordnet werden können. Entlang eines vorderen Randes 15 der Aufnahmeplatte sind Trägerplatten 36 angeordnet, auf denen beispielsweise Probenbehälterträger bereitgestellt werden können, die dann mittels einer automatischen Bestückungseinrichtung 38 (ggf. mit Barcodeleser oder dergleichen) auf die Aufnahmeplatte 12 (in Y-Richtung) bewegt werden können.

Der durch die Pipettiervorrichtung 30 nutzbare, insbesondere anfahrbare Bereich der Aufnahmeplatte 12 wird durch den hinteren Rand 13, den vorderen Rand 15 sowie die seitlichen Ränder 17 gebildet. Die Pipettiervorrichtung 30 kann insbesondere über den rechten Rand 17 in X-Richtung hinaus bewegt werden, damit hinter einem Trennelement 40, Einweg-Pipettierspitzen in einen an einer Halterung 42 vorgesehenen Müllbehälter (nicht dargestellt) entsorgt werden können.

Beim Probenverarbeitungssystem 10 sind am linken Rand 17 der Aufnahmeplatte beispielhaft und rein schematisch zwei Thermocycler 50, 52 dargestellt. Die Thermocycler 50, 52 sind im nutzbaren Bereich der Aufnahmeplatte 12 angeordnet. Die Thermocycler 50, 52 verfügen über je ein Verschlusselement 54, das vorzugsweise parallel zur Aufnahmeplatte 12 zwischen einer Öffnungsstellung und einer Schließstellung beweglich ist. Für den Thermocycler 50 ist das Verschlusselement 54 in einer etwa halb geöffneten Stellung dargestellt. Der Thermocycler 52 zeigt das Verschlusselement 54 in seiner Schließstellung. In den Thermocyclern 50, 52 können nicht dargestellte Probenbehälterträger, wie etwa PCR-Platten in einem Innenraum 56 aufgenommen werden, um in zugehörigen Probenbehältern (Wells) enthaltene Proben beispielsweise im Rahmen eines PCR-Verfahrens zu verarbeiten.

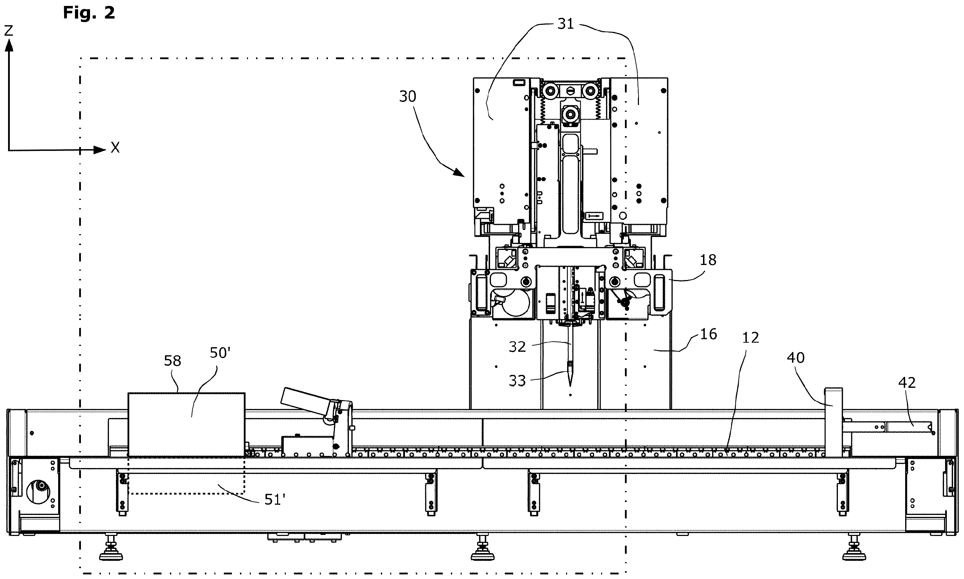

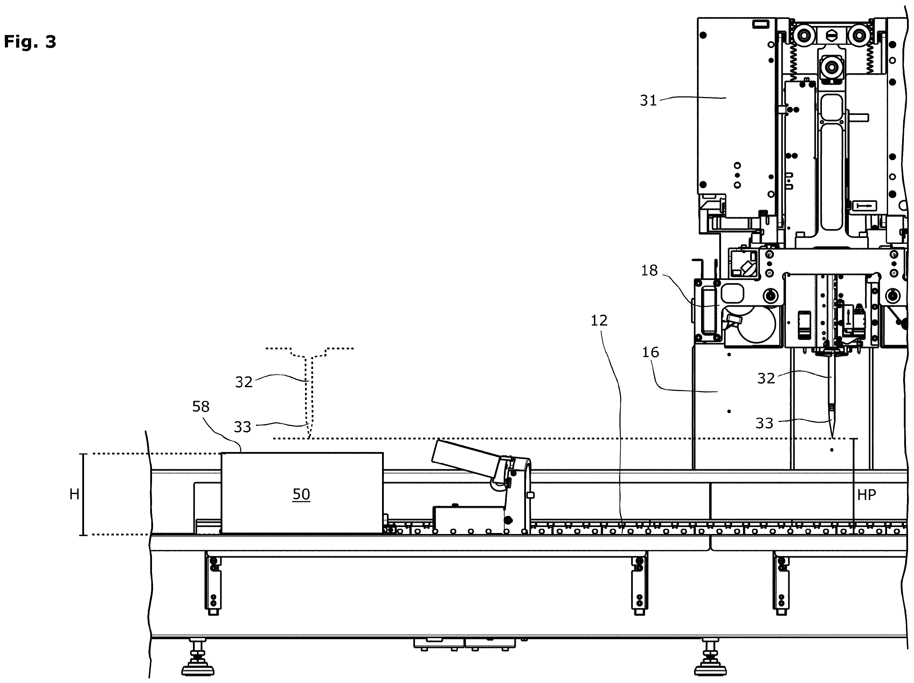

Unter Bezugnahme auf die Aufrissdarstellung der Fig. 2 und des vergrösserten Ausschnitts der Fig. 3 ist ersichtlich, dass der Thermocycler 50 einen höchsten Punkt 58 bzw. eine maximale Höhe H über der Aufnahmeplatte 12 aufweist. Diese Höhe (Abstand) H zwischen dem höchsten Punkt 58 bzw. der Oberseite des Thermocyclers 50 und der Aufnahmeplatte 12 ist kleiner als ein maximal möglicher Abstand HP eines unteren Endes einer Pipettierspitze 33, die an einem Pipettierkanal 32 befestigt ist. Hierdurch ist es möglich, dass mittels der Pipettiervorrichtung 30 Pipettiervorgänge an Probenbehältern vorgenommen werden können. die sich im Thermocycler 50 befinden. Der Abstand HP zwischen Aufnahmeplatte 12 und Pipettierspitze 33 kann auch als Überfahrhöhe bezeichnet werden. Die in der Fig. 3 dargestellten Abstände H und HP sind rein schematisch und beispielhaft. Der Unterschied zwischen diesen beiden Abständen kann auch kleiner sein. In der Regel wird davon ausgegangen, dass der Unterschied zwischen den Abständen H und HP wenige Millimeter beträgt, vorzugsweise weniger als 5 Millimeter. Der Thermocycler 50 bzw. 52 ist also derart an der Aufnahmeplatte angeordnet, dass die Pipettiervorrichtung 30 (mit Pipettierkanal 32 und Pipettierspitze 33, beide gestrichelt dargestellt) oberhalb von Probenbehältern, die sich im Thermocycler 50, 52 befinden, in eine einen Pipettiervorgang ermöglichende Stellung beweglich ist.

Es ist noch als vorteilhaft anzumerken, dass das Verschlusselement 54 durch seine im wesentlichen horizontale Bewegung in eine Öffnungsstellung nicht in den Bereich oberhalb der Aufnahmeplatte 12 gelangt, der größer als der Abstand HP ist. Die im Beispiel der Fig. 1 angedeutete Bewegungsrichtung des Verschlusselements 54 nach links (von der Aufnahmeplatte weg) ist insoweit von Vorteil, als dass das Verschlusselement in seiner Öffnungsstellung nicht in den nutzbaren Bereich der Aufnahmeplatte 12 hineinragt. Natürlich sind aber auch andere Bewegungsrichtungen, z.B in X-Richtung denkbar. Es kann auch an eine Art versenkbares Verschlusselement gedacht werden, das in mehrere zueinander bewegliche Segmente unterteilt ist und entlang dem Thermocyclergehäuse innerhalb oder ausserhalb des Gehäuses nach unten bewegt werden kann, um den Innenraum des Thermocyclers freizugeben. Die im Beispiel dargestellte einteilige Ausführung des Verschlusselements 54 ist ebenfalls nur optional. Das Verschlusselement kann auch durch zwei Teile gebildet sein, die jeweils den halben Innenraum abdecken können und die in entgegengesetzter oder gleicher Richtung bewegt und ggf. übereinander geschoben werden können. Das Verschlusselement 54 wird im übrigen vorteilhaft automatisch zwischen seiner Öffnungsstellung und seiner Schließstellung bewegt mittels einer entsprechenden Antriebskomponente.

Aus Fig. 2 ist ferner ersichtlich, dass auch ein Thermocycler 50' mit einer grösseren Bauhöhe verwendet werden kann. Ein derartiger Thermocycler müsste in einem nicht dargestellten Ausschnitt der Aufnahmeplatte 12 eingelassen werden, so dass ein gestrichelt dargestellter unterer Teil 51' des Thermocyclers 50' unterhalb der Aufnahmeplatte 12 angeordnet werden kann. Auch in einem solchen Falle ist es aber für das Probenverarbeitungssystem 10 besonders vorteilhaft, wenn der höchste Punkt 58 des Thermocyclers 50' einen geringeren Abstand H (Fig. 3) zur Aufnahmeplatte 12 aufweist als der Abstand HP (Fig. 3). Der maximale Abstand HP zwischen dem unteren Ende der wenigstens einen Pipettierspitze 33 und der Aufnahmeplatte 12 beträgt etwa 100mm bis 180mm, vorzugsweise etwa 125mm bis 150mm. Der höchste Punkt 58 bzw. die Oberseite des Thermocyclers 50, 50', 52 hat somit etwa einen Abstand H zur Aufnahmeplatte 12 von etwa 95mm bis 175mm, vorzugsweise etwa 120mm bis 145mm.

Um Probenbehälterträger (PCR-Platten) in einen Thermocycler zu bewegen bzw. aus diesem zu entfernen kann am Arbeitsarm 14 bzw. dem Ausleger 18 auch eine Greifeinrichtung (nicht dargestellt) vorgesehen sein. Eine derartige Greifeinrichtung kann beispielsweise durch zwei Greifer gebildet sein, die an Pipettierkanälen 32 angebracht werden können anstelle von Pipettierspitzen, so dass durch diese beiden Greifer-Pipettierkanäle ein Probenträgerbehälter ergriffen und transportiert werden kann. Sobald solche Transporte von Probenbehälterträger abgeschlossen sind, können die Greifer an einer bestimmten Position auf der Aufnahmeplatte in einer Bereitschaftsstellung angeordnet werden und die Pipettierkanäle können wieder Pipettierspitzen aufnehmen. Selbstverständlich kann auch eine von den Pipettierkanälen separate Greifeinrichtung eingesetzt werden. Denkbar ist auch eine außerhalb der Aufnahmeplatte angeordnete Greifeinrichtung, die aber den nutzbaren Bereich der Aufnahmeplatte wenigstens teilweise erreicht.

Nachfolgend werden Workflows I-III beschrieben, die eine PCR-Vorrichtung benötigen, bei welcher ein Verschlusselement (Deckel) 54 zu beliebigen Zeitpunkten während des PCR-Prozesses bzw. des Inkubations-Prozesses möglich ist. Bei diesen Workflows kann in vorteilhafter Weise ein Probenverarbeitungssystem 10, wie es beispielhaft in den Fig. 1 bis 3 dargestellt ist, zum Einsatz kommen. Der beschriebene Gesamtworkflow ist rein exemplarisch und umfasst eine PCR-Reaktion (Workflow I), einen Reinigungs-(Clean-Up-)Schritt (Workflow II) und ein Einrichten einer Sequenzierungs-Reaktion (Workflow III). Bei allen diesen Workflows werden die erforderlichen Pipettiervorgänge im Bereich des Thermocyclers 50, also an Probenbehältern im Thermocycler durchgeführt. Ein Entfernern der Probenbehälter bzw. Probenbehälterträger aus dem Thermocycler an eine andere Position zwecks Pipettieren ist nicht notwendig.Da das Verschlusselement 54 jederzeit während eines Temperaturschritts geöffnet werden kann, ist gemäß Workflow I beispielsweise eine sogenannte Hot-Start-PCR möglich. Auch ein Clean-Up-Prozess (Workflow II) kann direkt am Thermocycler ausgeführt werden, ohne dass die Probenbehälter bzw. der zugehörige Probenbehälterträger aus dem Thermocycler entfernt werden muss. Gemäß Workflow III kann ein Aliquot des gereinigten PCR-Produkts in andere Probenbehälter eines anderen Probenbehälterträgers pipettiert werden, wobei der andere Probenbehälterträger automatisch in einem anderen, im Bereich der Aufnahmeplatte angeordneten Thermocycler (z.B. Thermocycler 52) platziert worden ist. Die Zyklus-Sequenzierung-Reaktion wird dann pipettiert und das zugehörige Programm durchlaufen. Alternativ wird ein Aliquot des (nicht gereinigten) PCR- Produkts (nach Workflow I) direkt in andere Probenbehälter eines anderen Probenbehälterträgers in einem anderen Thermocycler pipettiert. Der Clean-Up-Prozess wird dann direkt im anderen Thermocycler durchgeführt mit anschliessendem Sequenzieren dieses gereinigten Aliquots im anderen Thermocycler.

Workflow I, als "Hot-Start PCR" bezeichnet, kann wie folgt ablaufen:
1. Verschlusselement (Deckel) 54 des Thermocyclers 50 wird geöffnet
2. Eine leere PCR-Platte (Probenbehälterträger) wird durch die automatische Greifeinrichtung in den geöffneten Thermocycler 50 platziert, der im Bereich der Aufnahmeplatte 12, sogenannt "on deck" angeordnet ist.
3. Hinzufügen von Probenflüssigkeit, PCR-Komponenten (Mastermix, Primer) mittels der Pipettiervorrichtung 30
4. Starten der ersten Phase des PCR-Reaktionsprofils bei 95°C für die Denaturierung
5. Wenn die Temperatur oberhalb der Annealing-Temperatur für den Primer liegt, wird die Taq-Polymerase oder irgendeine andere wesentliche Komponente für die PCR-Reaktionsmischung in die Probenbehälter (Wells) zugegeben mittels der Pipettiervorrichtung 30.
6. Typischerweise wird ein geringes Volumen (etwa 1-2 µl) in die Probenbehälter dispensiert. Aufgrund der hohen Temperatur der Flüssigkeit in den Probenbehältern, wird das Dispensieren durch ein auf dem Druck im Pipettierkanal 32 basierenden Steuer-/Regelsystem unterstützt (wie beispielsweise die von der Anmelderin bekannten MAD- oder ADC-Systeme), um das effiziente Hinzufügen (Dispensieren) der geringen Menge der Taq-Polymerase zu erreichen und um zu verhindern, dass beim Herausbewegen der Pipettierspitze 33 aus der Probenflüssigkeit keine Probenflüssigkeit in die (Einweg-)Pipettierspitze eindringen kann.
7. Der Denaturierungs-Prozess benötigt in der Regel 2 Minuten. Abhängig von der Konfiguration der Pipettiervorrichtung 30 mit Pipettierkanälen 32 soll in so viele Probenbehälter wie möglich gleichzeitig die Taq-Polymerase (oder irgendeine andere wesentliche Komponente für die PCR-Reaktionsmischung) dispensiert werden. Idealerweise kommt eine PCR-Platte (Probenbehälterträger) mit 96 Wells (Probenbehälter) zum Einsatz, wobei eine Pipettiervorrichtung 30 verwendet wird mit 8 Pipettierkanälen 32.
8. Nach diesem Pipettierschritt wird die PCR-Platte (Probenbehälterträger mit den Probenbehältern) mit einer Dichtungsmatte (Dichtungselement) abgedeckt und das Verschlusselement des Thermocyclers wird automatisch geschlossen.
9. Die Zyklen des PCR-Prozesses werden durchgeführt, beispielsweise wird 30 mal folgendes Temperaturprofil durchlaufen: 15 Sekunden 95°C, gefolgt von 30 Sekunden bei Annealing-Temperatur, die abhängig vom verwendeten Primer ist, und gefolgt von einem Schritt von 72°C die Polymerisation während einer Minute pro 1kb (1000 Basenpaare) zu polymerisierende Nukleinsäure-Sequenz.

Workflow II, bezeichnet als "Removal of unincorporated dNTP and Primer by Exo SAP IT", kann wie folgt beschrieben werden:
1. Nachdem die PCR-Reaktion beendet worden ist, wird das Verschlusselement 54 geöffnet und das Dichtungselement wird entfernt.
2. Es wird Exo SAP IT (eingetragene Marke der Affymetrix Inc) verwendet, um nicht eingebundene Primer und dNTPs zu zerstören. Pro 5µl PCR-Reaktionsflüssigkeit wird 2µl Exo SAP IT in jeden Probenbehälter hinzugefügt mittels der Pipettiervorrichtung 30.
3. Das Dichtungselement wird auf die PCR-Platte (Probenbehälterträger) platziert und das Verschlusselement 54 des Thermocyclers 50 wird geschlossen.
4. Inkubation bei 37°C während 15 Minuten zum Reinigen (Clean-Up) der PCR.
5. Inkubation bei 80°C während 15 Minuten zum Zerstören des Exo SAP IT Enzyms.
6. Abkühlen der Reaktion.

Workflow III kann wie folgt ablaufen:
1. Nach dem Reinigen (Clean-Up) wird das Verschlusselement 54 des Thermocyclers 50 geöffnet und das Dichtungselement wird entfernt.
2. Eine benötigte Menge des PCR-Produkts wird in eine andere, leere PCR-Platte pipettiert, die sich ein einem zweiten an/auf der Aufnahmeplatte 12 angeordneten Thermocycler 52 befindet.
3. Die Zyklus-Sequenzierungs-Reaktionsmischung "BigDye Terminator" (eingetragene Marke der Firma life technologies) wird auf die entnommenen Proben (Muster) pipettiert.
4. Ein Dichtungselement wird auf der PCR-Platte angeordnet und das Verschlusselement 54 des zweiten Thermocyclers 52 wird geschlossen.
5. Das Zyklus-Sequenzierungs-Programm wird durch den zweiten Thermocycler 52 durchgeführt.

Abschließend wird darauf hingewiesen, dass das Probenverarbeitungssystem 10 eine nicht dargestellte und auch nicht näher beschriebene Steuereinrichtung umfasst, wie etwa ein Computer, durch welche alle Bewegungen der Pipettiervorrichtung und einer Greifeinrichtung angesteuert werden können. Ferner kann die Steuereinrichtung auch den bzw. die Thermocycler 50, 50', 52 ansteuern, um gewünschte Zyklen zu fahren. Alternativ kann die Steuereinrichtung über eine entsprechende Schnittstelle mit einer internen Steuerung/Regelung der Thermocycler verbunden sein. Die beweglichen Teile, wie etwa Arbeitsarm, Pipettiervorrichtung, Pipettierkanäle, Greifeinrichtung, Verschlusselement der Thermocycler und dergleichen werden mittels geeigneter Antriebsmittel bewegt, wobei die Antriebsmittel ebenfalls durch die Steuereinrichtung angesteuert werden können.

Das beschriebene Probenverarbeitungssystem ermöglicht eine integrierte Durchführung von PCR-Verfahren inklusive aller Pipettiervorgänge, die während des PCR-Prozesses durchgeführt werden müssen. Dabei können im PCR-Prozess befindliche Probenbehälter im Thermocycler verweilen, wenn Pipettierschritte erforderlich sind. Das gesamte Verfahren kann somit stärker automatisiert und effizienter durchgeführt werden.

## Patentansprüche

1. Probenverarbeitungssystem zum Verarbeiten von chemischen oder biologischen Proben, insbesondere Körperflüssigkeiten wie Blut, Speichel, Sekrete und dergleichen oder Gewebeproben, insbesondere zur Verarbeitung von Proben für eine DNA-Analyse mittels PCR, umfassend
eine Dosiervorrichtung
mit einer Aufnahmeplatte (12), wobei die Aufnahmeplatte (12) in einer durch eine erste und eine zweite Richtung (X, Y) definierten Ebene im Wesentlichen horizontal angeordnet ist;
mit wenigstens einem Arbeitsarm (14, 16, 18), der relativ zur Aufnahmeplatte (12), vorzugsweise entlang einem ersten Rand (13) der Aufnahmeplatte (12), in der ersten Richtung (X) beweglich ist, und wobei sich der Arbeitsarm (14, 16, 18) in der zweiten Richtung (Y) über die Aufnahmeplatte (12) erstreckt, wobei am Arbeitsarm (14, 16, 18) wenigstens eine in der zweiten Richtung (Y) bewegliche Pipettiervorrichtung (30) angebracht ist, wobei die Pipettiervorrichtung (30) wenigstens einen in einer zur ersten und zweiten Richtung (X, Y) orthogonalen dritten Richtung (Z) beweglichen Pipettierkanal (32) mit einer daran angebrachten oder anbringbaren Pipettierspitze (33) umfasst und wobei die Pipettiervorrichtung (30) dazu eingerichtet ist, Flüssigkeit in bzw. aus einem Probenbehälter zu dispensieren bzw. zu aspirieren;
und
wenigstens einen Thermocycler (50, 52), der im Bereich der Aufnahmeplatte (12) vorgesehen ist,
**dadurch gekennzeichnet, dass** der Thermocycler (50, 52) derart an der Aufnahmeplatte (12) angeordnet ist, dass die Pipettiervorrichtung (30) oberhalb von Probenbehältern, die sich im Thermocycler (50, 52) befinden, in eine einen Pipettiervorgang ermöglichende Stellung beweglich ist.

2. Probenverarbeitungssystem nach Anspruch 1, **dadurch gekennzeichnet, dass** sie ferner eine Steuereinrichtung umfasst, die dazu eingerichtet ist, die Funktionen der Dosiervorrichtung, wie etwa Bewegung in den drei Richtungen oder/und Aspirieren bzw. Dispensieren von Flüssigkeit mittels der Pipettiervorrichtung (30), und die Funktionen des Thermocyclers (50, 52), wie etwa Durchführung von Zyklen im Rahmen eines PCR-Prozesses, anzusteuern bzw. zu regeln.

3. Probenverarbeitungssystem nach Anspruch 1 oder 2, **dadurch gekennzeichnet, dass** der Thermocycler (50, 52) auf der Aufnahmeplatte (12) angeordnet ist, wobei ein höchster Punkt (58) des Thermocyclers unterhalb eines maximal möglichen Abstands (HP) zwischen einem unteren Ende der wenigstens einen Pipettierspitze (33) und der Aufnahmeplatte (12) liegt, wobei der Abstand (HP) durch eine Bewegung der Pipettiervorrichtung (30) bzw. des Pipettierkanals (32) in der dritten Richtung (Z) ermöglicht wird.

4. Probenverarbeitungssystem nach Anspruch 1 oder 2, **dadurch gekennzeichnet, dass** der Thermocycler (50') in einem in der Aufnahmeplatte (12) ausgeführten Ausschnitt eingelassen ist, derart, dass eine Unterseite (51') des Thermocyclers (50') unterhalb der Aufnahmeplatte (12) liegt, wobei ein höchster Punkt (58) des Thermocyclers (50') unterhalb eines maximal möglichen Abstands (HP) zwischen einem unteren Ende der wenigstens einen Pipettierspitze (33) und der Aufnahmeplatte (12) liegt, wobei der Abstand (HP) durch eine Bewegung der Pipettiervorrichtung (30) bzw. des Pipettierkanals (32) in der dritten Richtung (Z) ermöglicht wird.

5. Probenverarbeitungssystem nach Anspruch 3 oder 4, **dadurch gekennzeichnet, dass** der maximale Abstand (HP) zwischen dem unteren Ende der wenigstens einen Pipettierspitze (33) und der Aufnahmeplatte (12) etwa 100mm bis 180mm, vorzugsweise etwa 125mm bis 150mm beträgt.

6. Probenverarbeitungssystem nach einem der vorhergehenden Ansprüche, **dadurch gekennzeichnet, dass** am Thermocycler (50, 52) ein deckelartiges Verschlusselement (54) zum Abdecken von im Thermocycler (50, 52) befindlichen Probenbehältern vorgesehen ist, wobei das Verschlusselement (54) im Wesentlichen parallel zur Aufnahmeplatte (12) relativ zum Thermocycler (50, 52) beweglich ist zwischen einer das Innere (56) des Thermocyclers (50) freigebenden Öffnungsstellung und einer das Innere (56) des Thermocyclers (54) abdeckenden Schließstellung.

7. Probenverarbeitungssystem nach Anspruch 6, **dadurch gekennzeichnet, dass** die Steuereinrichtung derart eingerichtet ist, dass das Verschlusselement (54) des Thermocyclers (50, 52) in Abhängigkeit der Relativstellung der Pipettiervorrichtung (30) zum Thermocycler (50, 52) in seine Öffnungs- oder Schließstellung bewegt wird, vorzugsweise dass das Verschlusselement (54) dann in seine Öffnungsstellung bewegt wird, wenn mittels der Pipettiervorrichtung (30) ein Pipettiervorgang an Probenbehältern im Thermocycler (50, 52) durchgeführt wird und dass das Verschlusselement (54) in seine Schließstellung bewegt wird, nachdem der Pipettiervorgang an Probenbehältern im Thermocycler (50, 52) beendet ist.

8. Probenverarbeitungssystem nach einem der vorhergehenden Ansprüche, ferner umfassend eine relativ zur Aufnahmeplatte (12) in der ersten, zweiten und dritten Richtung bewegliche Greifeinrichtung, die von der Steuereinrichtung angesteuert wird und dazu eingerichtet ist, Probenbehälterträger zu ergreifen und an gewünschte Positionen auf der Aufnahmeplatte (12) zu transportieren.

9. Probenverarbeitungssystem nach Anspruch 8, **dadurch gekennzeichnet, dass** die Greifeinrichtung dazu eingerichtet ist, Probenbehälterträger im bzw. aus dem Thermocycler (50, 52) abzusetzen bzw. zu entnehmen.

10. Probenverarbeitungssystem nach Anspruch 8 oder 9, **dadurch gekennzeichnet, dass** die Greifeinrichtung an dem Arbeitsarm (14, 16, 18) angeordnet ist oder an einem weiteren relativ zur Aufnahmeplatte (12) beweglichen Arbeitsarm vorgesehen ist.

11. Verfahren zum Steuern eines Probenverarbeitungssystems nach einem der vorhergehenden Ansprüche mittels seiner zugeordneten Steuereinrichtung, umfassend die Schritte:
Öffnen des Verschlusselements (54) eines ersten Thermocyclers (50);
Absetzen eines Probenbehälter enthaltenden Probenbehälterträgers im ersten Thermocycler (50) mittels der Greifeinrichtung, wobei die Probenbehälter mit Probenflüssigkeit befüllt oder unbefüllt sind;
Gegebenenfalls Befüllen der Probenbehälter mit zu analysierender Probenflüssigkeit mittels der Pipettiervorrichtung (30);
vorzugsweise Befüllen der Probenbehälter mit weiteren für die Analyse erforderlichen Komponenten, wie etwa Primer, Polymerase, Wasser und dergleichen mittels der Pipettiervorrichtung (30);
Durchführen eines PCR-Prozesses, wobei in Abhängigkeit der durchzuführenden Schritte des PCR-Prozesses das Verschlusselement (54) des ersten Thermocyclers (50) geöffnet oder geschlossen wird und wobei in Abhängigkeit der Stellung des Verschlusselements (54) weitere Komponenten in die im ersten Thermocycler (50) befindlichen Probenbehälter dispensiert werden mittels der Pipettiervorrichtung (30).

12. Verfahren nach Anspruch 11, ferner umfassend das vorzugsweise automatische Anordnen oder Entfernen eines die Probenbehälter abdichtenden Dichtungselements, wie etwa Dichtungsmatte oder dergleichen auf dem Probenbehälterträger in Abhängigkeit des PCR-Prozesses und der Stellung des Verschlusselements (54).

13. Verfahren nach Anspruch 11 oder 12, wobei nach Abschluss eines bestimmten Schritts des PCR-Prozesses das Verschlusselement (54) des ersten Thermocyclers (50) geöffnet wird, das vorzugsweise auf den Probenbehältern angeordnete Dichtungselement entfernt wird und aus den Probenbehältern prozessierte Probenflüssigkeit mittels der Pipettiervorrichtung (30) aspiriert wird und in weitere, insbesondere leere Probenbehälter außerhalb des ersten Thermocyclers (50) dispensiert wird, wobei die anderen Probenbehälter in einem Probenbehälterträger angeordnet sind, der vorzugsweise in einem zweiten insbesondere benachbarten Thermocycler (52) oder auf der Aufnahmeplatte (12) angeordnet ist.

14. Verfahren nach Anspruch 13, umfassend ferner das Dispensieren einer weiteren Komponente in die im ersten oder/und zweiten Thermocycler (50) enthaltenen Probenbehälter mittels der Pipettiervorrichtung, nach dem Pipettiervorgang das Anordnen des Dichtungselements auf den Probenbehältern, das Schließen des Verschlusselements (54) des ersten oder/und zweiten Thermocyclers (50) und das Durchführen von weiteren Schritten des PCR-Prozesses.
